# EUROPÄISCHE PATENTSCHRIFT

(11) **EP 0 901 813 B1**
(45) Veröffentlichungstag und Bekanntmachung des Hinweises auf die Patenterteilung: **04.12.2002**
(21) Anmeldenummer: 98117291.9
(22) Anmeldetag: 11.09.1998
(51) Int. Cl.: B01J 23/04, C07C 2/72

(54) **Katalysator und Verfahren zu Seitenkettenalkylierung**
Catalysts and side chain alkylation process
Catalyseur et procédé d'alkylation de chaines latérales

(30) Priorität: 15.09.1997 DE 19740539
(43) Veröffentlichungstag der Anmeldung: 17.03.1999
(73) Patentinhaber: BASF AKTIENGESELLSCHAFT, 67056 Ludwigshafen (DE)
(72) Erfinder: Narbeshuber, Thomas, Dr., 67069 Ludwigshafen (DE); Trefzer, Michael, 67166 Otterstadt (DE); Gehrer, Eugen, Dr., 67069 Ludwigshafen (DE)
(74) Vertreter: Isenbruck, Günter, Dr.

(56) Entgegenhaltungen:
- EP-A- 0 446 896
- EP-A- 0 558 941
- EP-A- 0 575 724
- EP-A- 0 636 597
- EP-A- 0 866 045

## Beschreibung

Die Erfindung betrifft einen Katalysator und ein Verfahren zur Seitenkettenalkylierung oder Seitenkettenalkenylierung von alkylaromatischen oder alkylalicyclischen Verbindungen mit Olefinen oder Diolefinen.

Die Seitenkettenalkylierung oder -alkenylierung, insbesondere von aromatischen Verbindungen, die ein azides Proton in der α-Position der Seitenkette tragen, in Gegenwart von Katalysatoren ist bekannt.

In der GB-B-902,043 ist ein geträgerter Alkalimetallkatalysator beschrieben. Der Katalysator besteht aus einem Alkalimetall auf Magnesiumoxid, Calciumoxid, Zinkoxid oder Bariumoxid als Träger. Der dotierte Träger wird calciniert und sodann mit dem Alkalimetall unter inerter Atmosphäre auf 300°C unter Rühren erhitzt. Der Katalysator wird beispielsweise zur Seitenkettenalkylierung von Toluol mit Ethen oder Propen eingesetzt.

EP-B-0 446 896 betrifft ein Verfahren zur Herstellung von alkylsubstituierten aromatischen Kohlenwasserstoffen. Als Katalysatoren werden metallisches Natrium oder Kalium auf Magnesiumoxid oder Calciumoxid geträgert eingesetzt. Das Magnesiumoxid oder Calciumoxid ist dabei wasserhaltig und wird durch Calcinieren eines entsprechenden Oxids oder Hydroxids hergestellt. Durch die Art der Ausführung des Calcinierens wird der Wassergehalt eingestellt. Als wasserfrei wird dabei ein Träger angesehen, der bei einer Temperatur von 800°C calciniert wurde. Die Katalysatoren werden beispielsweise zur Seitenkettenalkylierung von Toluol mit Ethen zu Isopropylbenzol eingesetzt.

Gemäß EP-B-0 575 724 wird ein Katalysator eingesetzt, der durch Tränken von Zirkoniumoxidpulver mit Kaliumhydroxidlösung, anschließendem Calcinieren in Luft bei 300 bis 700°C, gemäß den Beispielen bei 500°C und Aufbringen von metallischem Natrium auf den Träger hergestellt wird. Der Katalysator wird zur Seitenkettenalkenylierung von Toluol mit Butadien eingesetzt.

Gemäß EP-B-0 558 941 wird ein Katalysator eingesetzt, der durch Imprägnieren von Magnesiumoxidpulver mit einer wäßrigen Lösung von Kaliumcarbonat oder Kaliumhydroxid, Calcinieren in Luft bei 500°C und Aufbringen von metallischem Natrium hergestellt wird. Der Katalysator wird zur Seitenkettenalkenylierung von Xylol oder Ethylbenzol mit Butadien eingesetzt.

Gemäß EP-A-0 636 597 wird ein Verfahren zur Seitenkettenalkenylierung von o-Xylol mit Butadien beschrieben, wobei als Katalysator Aluminiumoxid, Calciumoxid oder Zirkoniumoxid verwendet wird, das mit wäßriger KOH imprägniert ist und danach bei einer Temperatur von 500 bis 550°C calciniert wurde. Der Träger wird mit metallischem Natrium beschichtet.

EP-A-0 866 045 (D2) betrifft ein Verfahren zur Herstellung einer monoalkenylaromatischen Kohlenwasserstoffverbindung durch Seitenkettenalkenylierung von aromatischen Kohlenwasserstoffen. Die Umsetzung wird in Gegenwart eines Katalysators durchgeführt, der hergestellt wird durch Calcinieren eines Gemisches eines Erdalkalimetallcarbonats oder - hydroxids und einer Kaliumverbindung. Nach dem Calcinieren wird ein Alkalimetall auf diesen Träger aufgebracht und das erhaltene Gemisch wird bei oder oberhalb von der Schmelztemperatur des Alkalimetalls für eine bestimmte Zeit wärmebehandelt. Das Erdalkalimetall kann beispielsweise Magnesium sein. Als Kaliumverbindung kann Kaliumhydroxid, Kaliumcarbonat oder Kaliumhydrogencarbonat eingesetzt werden. Das Alkalimetall kann Natrium sein.

Die Calcinierung des Katalysators wird bei einer Temperatur von 250 bis 700°C, vorzugsweise 350 bis 600°C durchgeführt, siehe Seite 3, Zeilen 53 bis 54. In den Beispielen wird die Calcinierung bei 500°C durchgeführt. Bei diesem Dokument handelt es sich um eine prioritätsältere, nicht vorveröffentlichte Anmeldung (Art. 54(3) (4) EPÜ).

Die bekannten Katalysatoren weisen in der Regel eine hohe Selektivität auf. Die katalytische Aktivität, die in den erreichbaren Umsätzen sichtbar wird, ist jedoch in vielen Fällen noch zu gering. Zudem ist bei den bekannten Katalysatoren eine Kalzinierung der Materialen bei hohen Temperaturen von etwa 500°C notwendig, um eine ausreichende Aktivität und Selektivität der Katalysatoren zu erhalten. Ein solches Herstellungsverfahren ist kostspielig.

Aufgabe der vorliegenden Erfindung ist die Bereitstellung eines Katalysators für die Seitenkettenalkylierung oder Seitenkettenalkenylierung, der die Nachteile der bekannten Katalysatoren vermeidet und neben einer hohen Selektivität eine hohe Aktivität und Standzeit aufweist und kostengünstig hergestellt werden kann.

Die Aufgabe wird erfindungsgemäß gelöst durch Bereitstellung eines Katalysators, enthaltend mindestens ein Alkalimetall auf einem dotierten Träger, wobei der dotierte Träger herstellbar ist aus mindestens einer Zirkonium- und gegebenenfalls zusätzlich Erdalkalimetallverbindung als Träger und mindestens einer Alkalimetallverbindung als Dotierung und Calcinieren des dotierten Trägers bei einer Temperatur von 250 bis 285°C, wobei das Gewichtsverhältnis Alkalimetall/Träger 0,01 bis 5 und das Gewichtsverhältnis Dotierung/Träger 0,01 bis 5 beträgt.

Die Aufgabe wird ferner gelöst durch Bereitstellung eines Verfahrens zur Seitenkettenalkylierung oder Seitenkettenalkenylierung von alkylaromatischen oder alkylalicyclischen Verbindungen durch Umsetzung mit Olefinen oder Diolefinen, wobei die Umsetzung in Gegenwart eines wie vorstehend definierten Katalysators durchgeführt wird.

Bei der Seitenbettenalkylierung kann der Katalysator-Träger auch aus mindestens einer Erdalkalimetallverbindung herstellbar sein.

Erfindungsgemäß wurde gefunden, daß insbesondere Katalysatoren auf Basis von mit Alkalimetallverbindungen dotierten Zirkonium- und/oder Erdalkalimetalloxiden als Träger eine deutlich höhere Aktivität und damit einen deutlich verbesserten Umsatz zeigen, wenn sie bei Temperaturen von 250 bis 285°C, calciniert wurden.

Im erfindungsgemäß eingesetzten Katalysator beträgt das Verhältnis Alkalimetall/Träger vorzugsweise 0,01 bis 2, besonders bevorzugt 0,01 bis 1. Das Alkalimetall ist dabei vorzugsweise Natrium oder Kalium, insbesondere Natrium. Auch Gemische mehrerer Alkalimetalle können eingesetzt werden.

Das Alkalimetall liegt auf einem dotierten Träger vor, der herstellbar ist und vorzugsweise hergestellt wird durch Zusammenbringen, insbesondere inniges Vermischen mindestens einer Zirkonium- und/oder Erkalkalimetallverbindung als Träger mit mindestens einer Alkalimetallverbindung als Dotierung und Calcinieren des dotierten Trägers bei den vorstehend angegebenen Temperaturen.

Die Herstellung des dotierten Trägers erfolgt vorzugsweise durch Vermischen wäßriger Lösungen und Ausfällen des dotierten Trägers, Vermischen der pulverförmigen Komponenten oder Imprägnieren des festen Trägers mit einer wäßrigen Lösung der Dotierung. Dabei können alle geeigneten Zirkonium-, Erdalkalimetall- und Alkalimetallverbindungen eingesetzt werden, die beim Calcinieren vorzugsweise in die oxidische Form überführt werden können. Vorzugsweise erfolgt dabei die Herstellung des dotierten Trägers aus Oxiden, Hydroxiden und/oder Halogeniden als Träger und Dotierung.

Die Zirkonium-, Erdalkalimetall- und/oder Alkalimetallverbindungen können beispielsweise in Form von Lösungen in beliebigen geeigneten Lösungsmitteln, vorzugsweise wäßrigen Lösungsmitteln, insbesondere als wäßrige Lösungen eingesetzt werden.

Vorzugsweise wird zur Herstellung des dotierten Trägers ein Pulver, eine Paste oder eine Aufschlämmung mindestens eines Zirkonium- und/oder Erdalkalimetallhydroxids als Träger mit einer wäßrigen Lösung mindestens eines Alkalimetallhydroxids und/oder -carbonats als Dotierung imprägniert, getrocknet und dann calciniert.

Der Träger ist dabei vorzugsweise ausgewählt aus Verbindungen von Zirkonium und/oder Magnesium. Die Dotierung ist vorzugsweise ausgewählt aus Kaliumverbindungen, besonders bevorzugt Kaliumhydroxid oder Kaliumcarbonat. Insbesondere wird Kaliumhydroxid oder Kaliumcarbonat eingesetzt.

Vorzugsweise wird ein Pulver oder eine Paste von Zirkoniumtetrahydroxid oder Magnesiumhydroxid als Träger mit einer wäßrigen Lösung von Kaliumcarbonat (K₂CO₃) oder Kaliumhydroxid (KOH) als Dotierung imprägniert. Als Alkalimetall wird dabei vorzugsweise Natrium aufgebracht.

### Herstellung der Katalysatoren

Die erfindungsgemäß eingesetzten Katalysatoren werden hergestellt durch
(a) Zusammenbringen, insbesondere inniges Vermischen mindestens einer Zirkonium- und/oder Erdalkalimetallverbindung als Träger mit mindestens einer Alkalimetallverbindung als Dotierung, gegebenenfalls Trocknen des dotierten Trägers,
(b) Calcinieren des so erhaltenen dotierten Trägers und
(c) Aufbringen mindestens eines Alkalimetalls auf den calcinierten dotierten Träger durch
   - Trägerung des schmelzflüssigen Alkalimetalls, oder
   - Imprägnierung oder Tränkung des Trägers mit Lösungen eines Alkalimetallazids, Trocknen des Trägers und Zersetzung des Alkalimetallazids, oder
   - Aufdampfen des Alkalimetalls auf den Träger, oder
   - Imprägnierung oder Tränkung des Trägers mit ammoniakalischen Lösungen des Alkalimetalls und Entfernen des Ammoniaks.

Das Vermischen von Pulvern, Pasten oder Aufschlämmungen kann dabei mit bekannten Vorrichtungen, beispielsweise Knetern oder Rührern erfolgen.

Bei Verwendung von wäßrigen Lösungen der Komponenten wird das Wasser vor dem Calcinieren des dotierten Trägers vorzugsweise durch Trocknen entfernt. Es kann auch ohne vorherige Trocknung calciniert werden, wobei zu Beginn der Calcinierung das (wäßrige) Lösungsmittel entweicht.

Die Calcinierung des dotierten Trägers kann unter vermindertem Druck, unter Normaldruck oder unter erhöhtem Druck durchgeführt werden. Sie kann dabei sowohl in sauerstoffhaltiger Atmosphäre, als auch in Inertgasatmosphäre, wie unter Helium, Stickstoff oder Argon, oder unter Reaktivgasatmosphäre, wie unter Wasserstoff, Ammoniak, Kohlendioxid oder Kohlenmonoxid stattfinden.

Die Alkalimetalle werden in an sich bekannter Weise auf die calcinierten und dotierten Träger aufgebracht. Hierzu gehört die Trägerung im schmelzflüssigen Zustand bei einer Temperatur im Bereich von 100 bis 300°C, wie sie beispielsweise in GB-A-1 143 993 beschrieben ist. Dazu wird die entsprechende Menge des Alkalimetalls als Strang oder Block zum Träger gegeben und unter Erwärmen mit ihm vermischt. Dabei erfolgt die feine Verteilung des Alkalimetalls auf dem Träger. Ferner können die Alkalimetalle durch Imprägnierung mit Lösungen der Alkalimetallazide und anschließende thermische Zersetzung der Azide hergestellt werden. Ein entsprechendes Verfahren ist beispielsweise in FR-A-2 609 024 beschrieben. Weiterhin können die Alkalimetalle durch Aufdampfen auf den Träger aufgebracht werden. Dies geschieht in der Regel unter vermindertem Druck.

Weiterhin können die Träger mit ammoniakalischen Lösungen der Alkalimetalle imprägniert werden, und das Ammoniak kann anschließend verflüchtigt werden. Die Trägerung der Alkalimetalle findet dabei vorzugsweise unter vermindertem Druck oder unter Inertgasatmosphäre, wie unter Helium, Stickstoff, Wasserstoff oder Argon statt.

Die Katalysatoren werden zur Seitenkettenalkylierung oder Seitenkettenalkenylierung von alkylaromatischen oder alkylalicyclischen Verbindungen mit Olefinen oder Diolefinen eingesetzt.

Dabei wird die Umsetzung im allgemeinen bei einer Temperatur von -50 bis 400°C, vorzugsweise bei einer Temperatur von -20 bis 300°C, besonders bevorzugt 80 bis 250°C, insbesondere 100 bis 220°C und einem Druck von vorzugsweise 0,1 bis 200, besonders bevorzugt 1 bis 150, insbesondere 1 bis 100 bar durchgeführt.

Als alkylaromatische Verbindungen können dabei alle geeigneten Alkylaromaten eingesetzt werden. Sie können als aromatischen Kern beispielsweise einen Benzol- oder Naphthalinkern aufweisen. Weiterhin sind alkylalicyclische Verbindungen geeignet, in denen der cyclische Kern ein cyclischer Alkyl-, Alkenyl- oder Alkinylrest sein kann. Auch Reste, in denen mehrere der Ringstrukturen miteinander verknüpft sind, können eingesetzt werden. Die Ringstrukturen weisen in α-Position der Seitenkette ein azides Wasserstoffatom auf. Vorzugsweise weisen sie mindestens einen Alkylrest auf, der an die cyclische Struktur gebunden ist. Die Alkylreste können dabei eine beliebige Länge aufweisen und durch weitere Substituenten substituiert sein. Vorzugsweise werden als alkylaromatische Verbindungen Benzole, die durch 1 bis 6, vorzugsweise 1 bis 3, insbesondere 1 bis 2 C₁₋₂₀-, vorzugsweise C₁₋₃-Alkylreste substituiert sind, Naphthaline, die durch 1 bis 10, vorzugsweise 1 bis 5, besonders bevorzugt 1 bis 2 C₁₋₂₀-, vorzugsweise C₁₋₃-Alkylreste substituiert sind, und als alkylalicyclische Verbindungen Cyclopentene oder Cyclohexene eingesetzt, die durch 1 bis 5, vorzugweise 1 oder 2, beziehungsweise 1 bis 6, vorzugsweise 1 bis 3, insbesondere 1 oder 2 C₁₋₂₀-, vorzugsweise C₁₋₃-Alkylreste substituiert sind.

Die Olefine weisen bei der Alkylierung vorzugsweise 2 bis 20, besonders bevorzugt 2 bis 10, insbesondere 2 bis 5 C-Atome auf. Vorzugsweise werden Ethen, Propen, 1-Buten, 2-Buten, Isobuten, 1-Penten, 2-Penten, 2-Methyl-1-buten, 2-Methyl-2-buten und/oder 3-Methyl-1-buten eingesetzt. Besonders bevorzugt sind Ethen und Propen. Die Diolefine weisen bei der Alkenylierung vorzugsweise 4 bis 20, besonders bevorzugt 4 bis 10, insbesondere 4 bis 6 C-Atome auf. Besonders bevorzugt werden Butadien und/oder Isopren eingesetzt.

Besonders bevorzugt sind die Umsetzung von Toluol mit Ethen oder Propen zu Propylbenzol oder Isobutylbenzol, die Umsetzung von Cumol mit Ethen zu tert.-Amylbenzol und die Umsetzung von Xylolen mit Butadien zu 5-Tolylpentenen.

Die Umsetzung kann diskontinuierlich oder vorzugsweise kontinuierlich in der Flüssig- oder Gasphase, bevorzugt in der Flüssigphase durchgeführt werden. Dabei können die bekannten Vorrichtungen zur Durchführung des Verfahrens eingesetzt werden.

Nachstehend wird die Erfindung anhand von Beispielen näher erläutert.

### Beispiele

### Herstellungsbeispiele

### Katalysatoren A-G Referenz, H und I erfindungsgemäß

### Katalysator A:

Mg(OH)₂-Pulver (Merck) wurde mit 10 Gew.-% KOH (gelöst in H₂O) imprägniert. Die Suspension wurde zur Trockene eingedampft und das so erhaltene Pulver bei 500°C 16h im Luftstrom kalziniert. Das Material wurde dann im Vakuum bei 280°C 3h lang trocken gerührt. Zu diesem Pulver wurden 10 Gew.% metallisches Natrium zugefügt und bei 280°C dispergiert.

### Katalysator B:

Mg(OH)₂-Pulver (Merck) wurde mit 10 Gew.-% KOH (gelöst in H₂O) imprägniert. Die Suspension wurde zur Trockene eingedampft und das so erhaltene Pulver bei 250°C 16h im Luftstrom kalziniert. Das Material wurde dann im Vakuum bei 250°C 3h lang trocken gerührt. Zu diesem Pulver wurden 10 Gew. % metallisches Natrium zugefügt und bei 250°C dispergiert.

### Katalysator C:

Mg(OH)₂-Pulver (Merck) wurde mit 68 Gew.% K₂CO₃ (gelöst in H₂O) imprägniert. Die Suspension wurde zur Trockene eingedampft und das erhaltene Pulver bei 500°C 16h im Luftstrom kalziniert. Das Material wurde dann im Vakuum bei 280°C 3h lang trocken gerührt. Zu diesem Pulver wurden 10 Gew.% metallisches Natrium zugefügt und bei 280°C dispergiert.

### Katalysator D:

Mg(OH)₂-Pulver (Merck) wurde mit 68 Gew.% K₂CO₃ (gelöst in H₂O) imprägniert. Die Suspension wurde zur Trockene eingedampft und das so erhaltene Pulver bei 250°C 16h im Luftstrom kalziniert. Das Material wurde dann im Vakuum bei 250°C 3h lang trocken gerührt. Zu diesem Pulver wurden 10 Gew.% metallisches Natrium zugefügt und bei 250°C dispergiert.

### Katalysator E:

Mg(OH)₂-Pulver (Merck) wurde mit 10 Gew.% KOH (gelöst in H₂O) imprägniert. Die Suspension wurde zur Trockene eingedampft und das so erhaltene Pulver bei 250°C 1h im Luftstrom kalziniert. Das Material wurde dann im Vakuum bei 250°C 3h lang trocken gerührt. Zu diesem Pulver wurden 10 Gew.% metallisches Natrium zugefügt und bei 250°C dispergiert.

### Katalysator F:

Mg(OH)₂-Pulver (Merck) wurde mit 10 Gew.% KOH (gelöst in H₂O) imprägniert. Die Suspension wurde zur Trockene eingedampft und das so erhaltene Pulver bei 250°C 2h im Luftstrom kalziniert. Das Material wurde dann im Vakuum bei 250°C 3h lang trocken gerührt. Zu diesem Pulver wurden 10 Gew.% metallisches Natrium zugefügt und bei 250°C dispergiert.

### Katalysator G:

Mg(OH)₂-Pulver (Merck) wurde mit 10 Gew.% KOH (gelöst in H₂O) imprägniert. Die Suspension wurde zur Trockene eingedampft und das so erhaltene Pulver bei 250°C 5h im Luftstrom kalziniert. Das Material wurde dann im Vakuum bei 250°C 3h lang trocken gerührt. Zu diesem Pulver wurden 10 Gew.% metallisches Natrium zugefügt und bei 250°C dispergiert.

### Katalysator H:

Zr(OH)₄-Paste (BASF) wurde mit 46 Gew.% KOH (gelöst in H₂O) imprägniert. Die Suspension wurde zur Trockene eingedampft und das so erhaltene Pulver bei 500°C 16h im Luftstrom kalziniert. Das Material wurde dann im Vakuum bei 280°C 3h lang trocken gerührt. Zu diesem Pulver wurden 10 Gew.% metallisches Natrium zugefügt und bei 280°C dispergiert.

### Katalysator I:

Zr(OH)₄-Paste (BASF) wurde mit 46 Gew.% KOH (gelöst in H₂O) imprägniert. Die Suspension wurde zur Trockene eingedampft und das so erhaltene Pulver bei 250°C 16h im Luftstrom kalziniert. Das Material wurde dann im Vakuum bei 250°C 3h lang trocken gerührt. Zu diesem Pulver wurden 10 Gew.% metallisches Natrium zugefügt und bei 250°C dispergiert.

### Beispiele 1 bis 9

Die Katalysatoren A-I wurden mit 85g Toluol in einem druckfesten Reaktionsgefäß vorgelegt. Nach Zugabe von 20g Propen wurde das Reaktionsgefäß auf 160°C aufgeheizt und die Reaktionssuspension 12 Stunden lang gerührt. In den Beispielen 1 bis 8 wurden jeweils 10g Katalysator, im Beispiel 9 6g Katalysator verwendet. Die Ergebnisse sind in Tabelle 1 aufgeführt.

**Tabelle 1**

| Beispiel | Kat. | U_{Tol} | U_{Prop} | Y_{iBB(Tol)} | Y_{iBB(Prop)} | S_{iBB(Tol)} | S_{iBB(Prop)} |
|---|---|---|---|---|---|---|---|
| 1 | A | 27,3% | 58,7% | 24,4% | 43,3% | 89,5% | 73,7% |
| 2 | B | 35,4% | 80,3% | 31,4% | 57,2% | 88,6% | 71,3% |
| 3 | C | 21,4% | 44,3% | 19,2% | 34,6% | 89,9% | 78,0% |
| 4 | D | 37,7% | 74,6% | 33,3% | 49,4% | 88,5% | 66,1% |
| 5 | E | 29,5% | 67,9% | 25,9% | 50,1% | 87,9% | 73,7% |
| 6 | F | 25,4% | 55,5% | 22,7% | 44,1% | 89,3% | 79,5% |
| 7 | G | 22,9% | 60,9% | 20,0% | 40,3% | 87,5% | 66,1% |
| 8 | H | 14.5% | 33,4% | 12,9% | 23,2% | 88,7% | 69,5% |
| 9 | I | 14,7% | 30,0% | 13,3% | 23,4% | 90,1% | 78,0% |
| U_{Tol} = Umsatz bezogen auf Toluol | | | | | | | |
| U_{Prop} = Umsatz bezogen auf Propen | | | | | | | |
| Y_{iBB(Tol)} = Ausbeute an Isobutylbenzol, bezogen auf Toluol | | | | | | | |
| Y_{iBB(Prop)} = Ausbeute an Isobutylbenzol, bezogen auf Propen | | | | | | | |
| S_{iBB(Tol)} = Selektivität für Isobutylbenzol, bezogen auf Toluol | | | | | | | |
| S_{iBB(Prop)} = Selektivität für Isobutylbenzol, bezogen auf Propen | | | | | | | |

## Patentansprüche

1. Verfahren zur Seitenkettenalkylierung von alkylaromatischen oder alkylalicyclischen Verbindungen durch Umsetzung mit Olefinen, **dadurch gekennzeichnet, daß** die Umsetzung in Gegenwart eines Katalysators durchgeführt wird, enthaltend mindestens ein Alkalimetall auf einem dotierten Träger, wobei der dotierte Träger herstellbar ist aus mindestens einer Zirkonium- und/oder Erdalkalimetallverbindung als Träger und mindestens einer Alkalimetallverbindung als Dotierung und Calcinieren des dotierten Trägers bei einer Temperatur von maximal 250 bis 285°C, wobei das Gewichtsverhältnis Alkalimetall/Träger 0,01 bis 5 und das Gewichtsverhältnis Dotierung/Träger 0,01 bis 5 beträgt.

2. Katalysator, enthaltend mindestens ein Alkalimetall auf einem dotierten Träger, wobei der dotierte Träger herstellbar ist aus mindestens einer Zirkonium- und gegebenenfalls zusätzlich Erdalkalimetallverbindung als Träger und mindestens einer Alkalimetallverbindung als Dotierung und Calcinieren des dotierten Trägers bei einer Temperatur von 250 bis 285°C, wobei das Gewichtsverhältnis Alkalimetall/Träger 0,01 bis 5 und das Gewichtsverhältnis Dotierung/Träger 0,01 bis 5 beträgt.

3. Katalysator nach Anspruch 2, **dadurch gekennzeichnet, daß** die Herstellung des dotierten Trägers durch Vermischen wäßriger Lösungen der Komponenten und Ausfällen des dotierten Trägers, Vermischen der pulverförmigen Komponenten oder Imprägnieren des festen Trägers mit einer wäßrigen Lösung der Dotierung erfolgt.

4. Katalysator nach Anspruch 2 oder 3, **dadurch gekennzeichnet, daß** die Herstellung des dotierten Trägers aus Oxiden, Hydroxiden und/oder Halogeniden als Träger und Dotierung erfolgt.

5. Katalysator nach einem der Ansprüche 2 bis 4, **dadurch gekennzeichnet, daß** zur Herstellung des dotierten Trägers ein Pulver, eine Paste oder eine Aufschlämmung mindestens eines Zirkonium- und gegebenenfalls zusätzlich Erdalkalimetallhydroxids als Träger mit einer wäßrigen Lösung mindestens eines Alkalimetallhydroxids und/oder - carbonats als Dotierung imprägniert, getrocknet und dann calciniert wird.

6. Katalysator nach Anspruch 5, **dadurch gekennzeichnet, daß** zu seiner Herstellung ein Pulver oder eine Paste von Zirkoniumtetrahydroxid als Träger mit einer wäßrigen Lösung von K₂CO₃ oder KOH als Dotierung imprägniert und als Alkalimetall Natrium auf den dotierten Träger aufgebracht wird.

7. Verfahren zur Herstellung eines Katalysators nach einem der Ansprüche 2 bis 6 durch
(a) Zusammenbringen, insbesondere inniges Vermischen mindestens einer Zirkonium- und gegebenenfalls zusätzlich Erdalkalimetallverbindung als Träger mit mindestens einer Alkalimetallverbindung als Dotierung, gegebenenfalls Trocknen des dotierten Trägers,
(b) Calcinieren des so erhaltenen dotierten Trägers und
(c) Aufbringen mindestens eines Alkalimetalls auf den calcinierten, dotierten Träger durch
- Trägerung des schmelzflüssigen Alkalimetalls, oder
- Imprägnierung oder Tränkung des Trägers mit Lösungen eines Alkalimetallazids, Trocknen des Trägers und Zersetzung des Alkalimetallazids, oder
- Aufdampfen des Alkalimetalls auf den Träger, oder
- Imprägnierung oder Tränkung des Trägers mit ammoniakalischen Lösungen des Alkalimetalls und Entfernen des Ammoniaks.

8. Verfahren zur Seitenkettenalkenylierung von alkylaromatischen oder alkylalicyclischen Verbindungen durch Umsetzung mit Diolefinen, **dadurch gekennzeichnet, daß** die Umsetzung in Gegenwart eines Katalysators durchgeführt wird, wie er in einem der Ansprüche 2 bis 6 definiert ist.

9. Verfahren nach Anspruch 1 oder 8, **dadurch gekennzeichnet, daß** die Umsetzung bei einer Temperatur im Bereich von -50 bis 400°C und einem Druck im Bereich von 0,1 bis 200 bar durchgeführt wird.

10. Verfahren nach einem der Ansprüche 1, 8 oder 9, **dadurch gekennzeichnet, daß** als alkylaromatische Verbindungen Benzole, die durch 1 bis 6 C₁₋₂₀-Alkylreste substituiert sind, Naphthaline, die durch 1 bis 10 C₁₋₂₀-Alkylreste substituiert sind, und als alkylalicyclische Verbindungen Cyclopentene oder Cyclohexene, die durch 1 bis 5 beziehungsweise 1 bis 6 C₁₋₂₀-Alkylreste substituiert sind, eingesetzt werden.

## Claims

1. A process for side-chain alkylation of alkylaromatic or alkylalicyclic compounds by reaction with olefins, **characterized in that** the reaction is carried out in the presence of a catalyst comprising at least one alkali metal on a doped carrier, where the doped carrier can be prepared from at least one zirconium and/or alkaline earth metal compound as carrier and at least one alkali metal compound as dopant by calcining the doped carrier at not more than 250 to 285°C, the alkali metal/carrier ratio by weight being from 0.01 to 5 and the dopant/carrier ratio by weight being from 0.01 to 5.

2. A catalyst comprising at least one alkali metal on a doped carrier, where the doped carrier can be prepared from at least one zirconium and, if appropriate, additionally alkaline earth metal compound as carrier and at least one alkali metal compound as dopant by calcining the doped carrier at from 250 to 285°C, the alkali metal/carrier ratio by weight being from 0.01 to 5 and the dopant/carrier ratio by weight being from 0.01 to 5.

3. A catalyst as claimed in claim 2, **characterized in that** the doped carrier is prepared by mixing aqueous solutions of the components and precipitating the doped carrier, mixing the powdered components or impregnating the solid carrier with an aqueous solution of the dopant.

4. A catalyst as claimed in claim 2 or 3, **characterized in that** the doped carrier is prepared from oxides, hydroxides and/or halides as carrier and dopant.

5. A catalyst as claimed in any of claims 2 to 4, **characterized in that** the doped carrier is prepared by impregnating a powder, a paste or a suspension of at least one zirconium and, if appropriate, additionally alkaline earth metal hydroxide as carrier with an aqueous solution of at least one alkali metal hydroxide and/or carbonate as dopant, drying and then calcining.

6. A catalyst as claimed in claim 5, **characterized in that** it is prepared by impregnating a powder or a paste of zirconium tetrahydroxide as carrier with an aqueous solution of K₂CO₃ or KOH as dopant, and applying sodium as alkali metal to the doped carrier.

7. A process for preparing a catalyst as claimed in any of claims 2 to 6, by
(a) mixing, in particular mixing intimately, at least one zirconium and, if appropriate, additionally alkaline earth metal compound as carrier with at least one alkali metal compound as dopant, where appropriate drying the doped carrier,
(b) calcining the doped carrier obtained in this way and
(c) applying at least one alkali metal to the calcined doped carrier by
- applying the molten alkali metal to the carrier, or
- impregnating the carrier with solutions of an alkali metal azide, drying the carrier and decomposing the alkali metal azide, or
- vapor-deposition of the alkali metal on the carrier, or
- impregnating the carrier with ammoniacal solutions of the alkali metal and removing the ammonia.

8. A process for side-chain alkenylation of alkylaromatic or alkylalicyclic compounds by reacting with diolefins, **characterized in that** the reaction is carried out in the presence of a catalyst as defined in any of claims 2 to 6.

9. A process as claimed in claim 1 or 8, **characterized in that** the reaction is carried out at from -50 to 400°C under a pressure in the range from 0.1 to 200 bar.

10. A process as claimed in any of claims 1, 8 and 9, **characterized in that** the alkylaromatic compounds employed are benzenes substituted by 1 to 6 C₁₋₂₀-alkyl radicals or naphthalenes substituted by 1 to 10 C₁₋₂₀-alkyl radicals, and the alkylalicyclic compounds employed are cyclopentenes or cyclohexenes respectively substituted by 1 to 5 or 1 to 6 C₁₋₂₀-alkyl radicals.

## Revendications

1. Procédé pour l'alkylation de chaînes latérales de composés alkylaromatiques ou alkylalicycliques par réaction avec des oléfines, **caractérisé en ce que** la réaction est réalisée en présence d'un catalyseur, contenant au moins un métal alcalin sur un support dopé, le support dopé pouvant être préparé à partir d'au moins un composé du zirconium et/ou de métal alcalino-terreux comme support et d'au moins un composé de métal alcalin comme dopage et par calcination du support dopé à une température d'au maximum 250 à 285 °C, le rapport pondéral métal alcalin/support étant de 0,01 à 5 et le rapport pondéral dopage/support étant de 0,01 à 5.

2. Catalyseur contenant au moins un métal alcalin sur un support dopé, le support dopé pouvant être préparé à partir d'au moins un composé du zirconium et le cas échéant, de plus, un composé de métal alcalino-terreux comme support et d'au moins un composé de métal alcalin comme dopage et par calcination du support dopé à une température de 250 à 285 °C, le rapport pondéral métal alcalin/support étant de 0,01 à 5 et le rapport pondéral dopage/support étant de 0,01 à 5.

3. Catalyseur selon la revendication 2, **caractérisé en ce que** la préparation du support dopé est réalisée par mélange des solutions aqueuses des composants et précipitation du support dopé, par mélange des composants sous forme de poudre ou par imprégnation du support solide avec une solution aqueuse du dopage.

4. Catalyseur selon la revendication 2 ou 3, **caractérisé en ce que** la préparation du support dopé est réalisée à partir d'oxydes, d'hydroxydes et/ou d'halogénures comme support et dopage.

5. Catalyseur selon l'une quelconque des revendications 2 à 4, **caractérisé en ce que** pour la préparation du support dopé, une poudre, une pâte ou une suspension d'au moins un hydroxyde de zirconium et le cas échéant, de plus, d'un hydroxyde de métal alcalino-terreux comme support est imprégnée d'une solution aqueuse d'au moins un hydroxyde et/ou un carbonate de métal alcalin comme dopage, séchée puis calcinée.

6. Catalyseur selon la revendication 5, **caractérisé en ce que** pour sa fabrication, une poudre ou une pâte de tétrahydroxyde de zirconium comme support est imprégnée d'une solution aqueuse de K₂CO₃ ou KOH comme dopage et que du sodium est appliqué comme métal alcalin sur le support dopé.

7. Procédé pour la préparation d'un catalyseur selon l'une quelconque des revendications 2 à 6, par
(a) rassemblement, en particulier mélange intime, d'au moins un composé du zirconium et le cas échéant, de plus, un composé de métal alcalino-terreux comme support avec au moins un composé de métal alcalin comme dopage, le cas échéant séchage du support dopé,
(b) calcination du support dopé ainsi obtenu et
(c) application sur le support calciné dopé d'au moins un métal alcalin par
- support du métal alcalin fondu liquide ou
- imprégnation ou imbibition du support avec des solutions d'un azide de métal alcalin, séchage du support et décomposition de l'azide de métal alcalin ou
- dépôt par vaporisation du métal alcalin sur le support ou
- imprégnation ou imbibition du support avec des solutions ammoniacales du métal alcalin et élimination de l'ammoniaque.

8. Procédé pour l'alkylation de chaînes latérales de composés alkylaromatiques ou alkylalicycliques par réaction avec des dioléfines, **caractérisé en ce que** la réaction est réalisée en présence d'un catalyseur tel qu'il est défini dans les revendications 2 à 6.

9. Procédé selon la revendication 1 ou 8, **caractérisé en ce que** la réaction est réalisée à une température dans la plage de -50 à 400 °C et à une pression dans la plage de 0,1 à 200 bars.

10. Procédé selon l'une quelconque des revendications 1, 8 ou 9, **caractérisé en ce qu'**on utilise comme composés alkylaromatiques des benzènes qui sont substitués par 1 à 6 résidus alkyle en C₁ à C₂₀, des naphtalènes qui sont substitués par 1 à 10 résidus alkyle en C₁ à C₂₀, et comme composés alkylalicycliques des cyclopentènes ou des cyclohexènes qui sont substitués par 1 à 5, respectivement 1 à 6, résidus alkyle en C₁ à C₂₀.
